# FASCICULE DE BREVET EUROPEEN

(11) **EP 0 614 980 B1**
(45) Date de publication et mention de la délivrance du brevet: **13.04.2005**
(21) Numéro de dépôt: 94400005.8
(22) Date de dépôt: 03.01.1994
(51) Int. Cl.: A61K 39/21

(54) **Variants trans-dominants TAT du virus de l'immunodéficience humaine**
TAT transdominante Variante aus Human Immunodeficiency Virus
TAT transdominant variants from human Immunodeficiency virus

(30) Priorité: 04.01.1993 FR 9300004
(43) Date de publication de la demande: 14.09.1994
(60) Demande divisionnaire: 03025262.1
(73) Titulaire: TRANSGENE S.A., 67000 Strasbourg (FR)
(72) Inventeur: Mehtali, Majid, F-67000 Strasbourg (FR); Sorg, Tania, F-67100 Strasbourg (FR)
(74) Mandataire: Warcoin, Jacques

(56) Documents cités:
- WO-A-89/12461
- CELL. vol. 58, no. 1 , 14 Juillet 1989 , CAMBRIDGE, NA US pages 215 - 223 GREEN, M. ET AL. 'Mutational analysis of HIV-1 Tat minimal domain peptides: identification of trans-dominant mutants that suppress HIV-LTR-driven gene expression'
- NUCLEIC ACIDS RESEARCH. vol. 17, no. 9 , 1989 , ARLINGTON, VIRGINIA US pages 3551 - 3561 KUPPUSWAMY, M. ET AL. 'Multiple functional domains of Tat, the trans-activator of HIV-1, defined by mutational analysis'
- EMBO JOURNAL. vol. 7, no. 10 , Octobre 1988 , EYNSHAM, OXFORD GB pages 3143 - 3147 GARCIA, J. A. ET AL. 'Functional domains required for tat-induced transcriptional activation of the HIV-1 long terminal repeat'

### LISTE DE SEQUENCES

(1) INFORMATION GENERALE:
   (i) DEPOSANT:
      (A) NOM: Transgene S.A.
      (B) RUE: 11 rue de Molsheim
      (C) VILLE: Strasbourg
      (E) PAYS: France
      (F) CODE POSTAL: 67082
      (G) TELEPHONE: (33) 88 27 91 00
      (H) TELECOPIE: (33) 88 58 07
   (ii) TITRE DE L' INVENTION: Nouveaux variants trans-dominants TAT du virus de l'immunodeficience humaine.
   (iii) NOMBRE DE SEQUENCES: 7
   (iv) FORME LISIBLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.25 (OEB)
(2) INFORMATION POUR LA SEQ ID NO: 1:
   (i) CARACTERISTIQUES DE LA SEQUENCE:
      (A) LONGUEUR: 26 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de mutagenese (38Phe en Asp)
   (xi) DESCRIPTION DE LA SEQUENCE: SEQ ID NO: 1:
   TAAAGCTTTT GTTGTGTCAC AAACTT 26
(2) INFORMATION POUR LA SEQ ID NO: 2:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de mutagenese (TAT 41Lys en Ala)
   (xi) DESCRIPTION DE LA SEQUENCE: SEQ ID NO: 2:
   GCCTAAGGCT GCTGTTGTGA A 21
(2) INFORMATION POUR LA SEQ ID NO: 3:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de mutagenese (TAT 54Gln en stop)
   (xi) DESCRIPTION DE LA SEQUENCE: SEQ ID NO: 3:
   TCTTCGTCGT TATCTCCGCT T 21
(2) INFORMATION POUR LA SEQ ID NO: 4:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de mutagenese (TAT 40Thr en Ala)
   (xi) DESCRIPTION DE LA SEQUENCE: SEQ ID NO: 4:
   TAAAGCTTTT GCTGTGAAAC 20
(2) INFORMATION POUR LA SEQ ID NO: 5:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de mutagenese (TAT 41Lys en Glu)
   (xi) DESCRIPTION DE LA SEQUENCE: SEQ ID NO: 5:
   GCCTAAAGCT TCTGTTGTGA A 21
(2) INFORMATION POUR LA SEQ ID NO: 6:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonucleotide de mutagenese (TAT 45Ile en Ser)
   (xi) DESCRIPTION DE LA SEQUENCE: SEQ ID NO: 6:
   GCCATAGGAG GATCCTAAGG 20
(2) INFORMATION POUR LA SEQ ID NO: 7:
   (i) CARACTERISTIQUES DE LA SEQUENCE:
      (A) LONGUEUR: 18 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (C) INDIVIDUEL ISOLE: oligonuclectide de mutagenese (TAT 47Tyr en Arg)
   (xi) DESCRIPTION DE LA SEQUENCE: SEQ ID NO: 7:
   TCCTGCCTCT AGAGATGC 18

## Description

La présente invention a pour objet de nouveaux variants de la protéine TAT du virus de l'immunodéficience humaine (VIH), ainsi que les fragments d'ADN codant pour lesdits variants, les cassettes d'expression permettant leur expression par voie recombinante et les cellules contenant lesdites cassettes d'expression. Les variants de la protéine TAT, cassettes et cellules sont notamment utiles pour la prévention ou le traitement des infections à VIH.

Le syndrome d'immunodéficience acquise (SIDA) se développe à la suite d'une infection des lymphocytes T4 d'un individu par le virus VIH. L'infection peut être asymptomatique pendant de nombreuses années, mais dès que les cellules sont activées, le virus VIH se réplique rapidement et les détruit. Le SIDA se caractérise par un déficit de l'immunité cellulaire, qui a pour effet de rendre l'individu particulièrement sensible à toute infection opportuniste. En juillet 1992, l'OMS a enregistré 501 272 cas de SIDA dans le monde. (AIDS Information international literature on acquired immunodeficiency syndrom and related retroviruses, 1992, **8**, Leeds University Press. Editor A.W. Boylston, Leeds). Mais ces chiffres sont toutefois inférieurs à la réalité, puisque cette maladie constitue une véritable épidémie dans certains pays africains.

Le SIDA reste une maladie dont le taux de mortalité est toujours très élevé en 1992. En effet, 90% des personnes décèdent dans les 2 ans suivant l'apparition du SIDA. Jusqu'à présent, aucun traitement ne s'est révélé être totalement satisfaisant malgré les multiples efforts investis. Le développement de traitements efficaces s'est heurté à la complexité particulière du virus VIH.

Le VIH est un rétrovirus qui appartient à la famille des lentivirus. Comme tout rétrovirus, le VIH est formé d'une enveloppe entourant une capside de nature protéique, qui contient le matériel génétique constitué par une molécule d'ARN associée à diverses protéines virales nécessaires aux premières étapes du cycle réplicatif.

Après infection d'un lymphocyte T, la molécule d'ARN est copiée par la reverse-transcriptase virale en ADN. L'ADN s'intègre dans le génome cellulaire et constitue ce qui est couramment appelé un provirus. L'ADN proviral peut y demeurer à l'état latent ou peut être transcrit en ARN par la machinerie cellulaire pour produire d'une part l'ARN génomique viral et d'autre part les ARNs messagers (ARNm) qui seront traduits en protéines virales.

La formation des nouvelles particules virales ou virions s'effectue par encapsidation de l'ARN génomique viral dans la capside. La particule ainsi formée se détache de la cellule par bourgeonnement en entraînant une partie de la membrane cellulaire, dans laquelle est incorporée la glycoprotéine d'enveloppe virale. Les virions ainsi libérés sont susceptibles d'infecter d'autres cellules lymphoïdes grâce à une reconnaissance spécifique et réciproque du récepteur CD4 exprimé à la surface des lymphocytes T4 et de la protéine d'enveloppe du VIH.

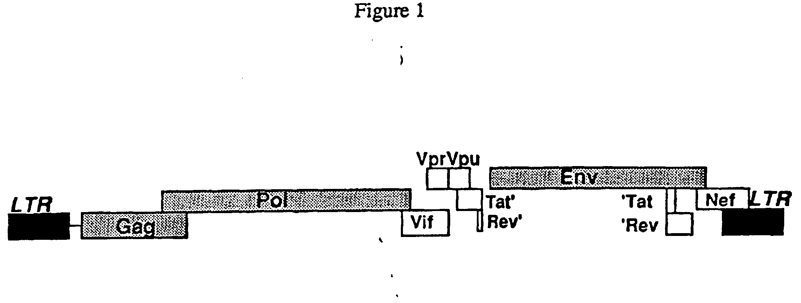

D'une manière générale, le génome du VIH comprend, comme indiqué dans la Figure 1 :
3 gènes structuraux : *gag, pol* et *env* codant respectivement pour les protéines de la capside, l'enveloppe et la reverse-transcriptase,
au moins 6 gènes : *tat, rev, nef, vif, vpr* et *vpu,* qui codent pour des protéines ayant vraisemblablement des fonctions régulatrices encore mal définies pour certaines et,
les séquences cis-régulatrices indispensables à la transcription, localisées aux 2 extrémités 5' et 3' de l'ADN proviral au niveau des LTRs (Long Terminal Repeat). Le LTR 5' contient les séquences promotrices et les éléments de régulation requis pour l'initiation de la transcription, alors que le LTR 3' est impliqué dans la terminaison de la transcription. En outre, la transcription des gènes viraux est soumise à une régulation complexe contrôlée notamment par les protéines virales TAT et REV.

La protéine TAT est une protéine régulatrice exprimée précocement durant le cycle viral. Son lieu d'action est le noyau. Son rôle consiste à trans-activer l'expression des gènes codant pour toutes les protéines du VIH. La protéine TAT interagit spécifiquement avec une courte séquence nucléotidique du génome viral : la séquence TAR (Trans-Activation Responsive Region) localisée à l'extrémité 5' du génome du VIH entre les nucléotides (nt) -17 à +80 du LTR 5'. Cette séquence est donc en partie présente dans tous les ARNm viraux. On suppose que le complexe TAT-TAR, probablement en association avec d'autres facteurs cellulaires, favorise la transcription des gènes viraux, stabilise les ARNm ainsi obtenus et améliore la traduction de ces ARNm en protéines. Ainsi le virus se multiplie très rapidement dès que le gène *tat* est activé.

L'effet de trans-activation induit par la protéine TAT a pu être évalué in vitro en utilisant un gène indicateur dont l'expression peut être facilement mesurée, tel que le gène CAT (Chloramphénicol Acetyl Transférase). Le gène CAT, placé sous le contrôle du LTR 5' du virus VIH incluant la séquence TAR, a été introduit dans des cellules animales par transfection. Dans les cellules qui n'expriment pas TAT, par exemple des cellules non infectées par le virus VIH, la transcription du gène CAT à partir du promoteur viral est bloquée ou réduite à un minimum, de sorte que pas ou très peu de produit du gène CAT est mis en évidence. Alors qu'en présence de la protéine TAT, on observe une augmentation de l'expression génique d'un facteur 100 à 1000 selon les cellules, qui résulte d'une accélération de la transcription associée à une meilleure efficacité de la traduction.

Le gène trans-activateur *tat* du VIH comprend 2 exons codants. Le premier est situé dans la région centrale du génome, entre les séquences codant pour les gènes *pol* et *env,* et code pour l'essentiel de la protéine, à savoir les 72 acides aminés N-terminaux. Le second, qui ne code que pour les 14 acides aminés C-terminaux n'est pas indispensable à l'activité biologique.

La structure de la protéine TAT est suggérée par sa fonction. Elle doit comprendre au moins un domaine qui permet l'activation de la transcription des gènes viraux, dit domaine d'activation, un domaine de liaison à la séquence nucléotidique TAR, sa séquence cible, et un domaine qui permet sa localisation nucléaire.

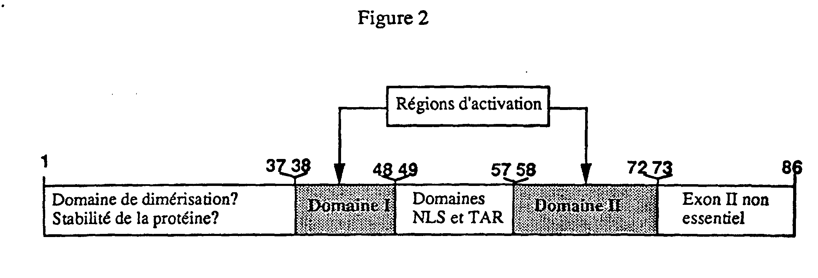

De nombreuses études ont montré que la protéine TAT est constituée de 5 domaines (Fig 2) :
un premier domaine (acides aminés 1 à 37) possède une séquence riche en cystéines, dont la fonction est encore inconnue. Certaines études suggèrent que cette région intervient dans le repliement de la protéine et dans la dimérisation des molécules TAT et la protégerait vis à vis des protéases,
le domaine constitué des acides aminés 38 à 48 correspond au premier domaine d'activation,
le domaine constitué des acides aminés 49 à 57 possède les séquences de localisation nucléaire et de fixation à l'élément TAR,
le domaine constitué des acides aminés 58 à 72 correspond au deuxième domaine d'activation de TAT,
le domaine constitué des acides aminés 73 à 86 est non essentiel à l'activité de TAT.

La plupart des rétrovirus apparentés au VIH possèdent des gènes trans-activateurs codant pour des protéines capables d'activer la transcription des gènes viraux à partir des LTRs. Depuis quelques années, de nombreux variants de ces protéines trans-activatrices dérivés de différents types de virus ont été décrits, et parmi eux des variants négatifs et dominants (Wachsman et al., Science, 1987, **235**, 674-677 ; Friedman et al., Nature, 1988, **335**, 452-454), désignés ci-après variants trans-dominants.

Les variants trans-dominants ont été définis comme des variants qui ont une capacité réduite à induire l'activation de l'expression des gènes placés sous le contrôle du promoteur viral (activité trans-activatrice réduite) mais qui ont la capacité de reconnaître leur séquence cible située au niveau de ce même promoteur, de sorte qu'ils peuvent inhiber de façon compétitive la fonction de la protéine trans-activatrice native.

Puisque ces variants interfèrent d'une manière dominante avec la fonction des protéines natives, ils pourraient constituer une nouvelle classe d'agents anti-viraux capables de promouvoir l'"immunisation intracellulaire" des cellules infectées.

D'une manière générale, cette technologie consiste à modifier génétiquement des cellules afin de leur faire synthétiser une protéine ou des acides nucléiques leur conférant un avantage particulier, comme par exemple selon le concept défini par D. Baltimore (Nature, 1988, **335**, 395-396), une résistance contre l'infection par un virus donné, tel que le virus VIH.

Ainsi, Green et al. (Cell, 1989, **58**, 215-233) ont généré des variants trans-dominants modifiés dans le premier domaine d'activation de la protéine TAT du virus VIH. Ces auteurs divulguent 4 variants issus d'un fragment peptidique de la protéine TAT : deux d'entre eux sont décrits comme étant des trans-dominants efficaces, à savoir les variants TAT (Lys⁴¹ -> Ala) et TAT (Tyr⁴⁷ -> Ala) et les deux autres des trans-dominants modérés, à savoir TAT (Ser⁴⁶ -> Ala) et le double variant TAT (Ser⁴⁶, Tyr⁴⁷ -> Ala, Ala).

Cependant, les variants mentionnés dans cette publication semblent controversés (Frankel et al., Proc. Natl. Acad. Sci. USA, 1989, **86**, 7397-7401). La demanderesse a également essayé de reproduire les résultats obtenus avec le variant (Lys⁴¹ - > Ala), mais sans succès, comme indiqué ci-après.

D'autre part, Pearson et al. (Proc. Natl. Acad. Sci. USA, 1990, **87**, 5079-5083) met en évidence l'importance de la région constituée des acides aminés 48 à 54 dans l'obtention d'un phénotype trans-dominant. En effet, le remplacement du codon codant pour la glutamine (Gln) en position 54 par un codon stop génère un variant tronqué ΔTAT qui présente un tel phénotype.

On a maintenant trouvé de nouveaux variants de la protéine TAT du VIH qui présentent un phénotype trans-dominant et qui ont été modifiés au niveau de certains résidus des domaines d'activation.

Ces variants sont notamment utiles dans le cadre d'une thérapie anti-SIDA, puisqu'ils présentent une activité trans-activatrice réduite et qu'ils interfèrent de manière dominante avec la fonction de la protéine TAT native. Ces nouveaux variants pourraient constituer des agents anti-viraux efficaces pour inhiber la réplication et la propagation du virus VIH. En effet, l'action de ces variants trans-dominants se ferait dès le premier cycle d'infection avant même que le virus n'ait pu synthétiser les protéines et l'ARN nécessaires à la formation de nouvelles particules virales.

La présente invention a donc pour objet un variant trans-dominant de la protéine TAT du virus VIH ou d'un fragment fonctionnel de ladite protéine comprenant au moins une mutation ; ladite mutation étant caractérisée par la présence d'un résidu acide aminé différent du résidu naturel en position 38.

D'une manière générale, on convient de décrire la séquence d'un variant de la protéine TAT sur la base de la séquence de la protéine TAT du virus VIH1 isolat Lai, telle que divulguée par Wain-Hobson et al. (Cell, 1985, **40**, 9-17).

Cependant, la protéine TAT et le fragment d'ADN qui code pour la protéine TAT ont été originairement décrits à partir de la souche virale VIH 1, isolat Lai. Or les souches virales et les isolats viraux décrits à l'intérieur d'une même souche existent en grand nombre. De plus, un virus particulier est susceptible de variabilité lors de sa propagation. Par conséquent, le fragment d'ADN qui code pour la protéine TAT peut avoir une séquence nucléotidique différente d'un virus à l'autre. De même, la protéine TAT peut avoir une séquence en acides aminés différente d'un virus à l'autre. Mais le dénominateur commun est la fonction de la protéine qui est de trans-activer l'expression des gènes placés sous le contrôle d'un promoteur contenant une séquence cible TAR, tel que le promoteur du virus VIH inclus dans le LTR 5' du génome viral. Par protéine TAT du VIH, on entend toute protéine donnant un résultat de trans-activation substantiellement identique à celui présenté par la protéine TAT du virus VIH1 isolat Lai.

En pratique, la séquence d'un variant de la protéine TAT est alignée sur celle de la protéine TAT du virus VIH1. La numérotation des acides aminés dans la séquence d'un variant de la protéine TAT sera donc calquée sur celle établie pour la protéine TAT du virus VIH1. Ainsi un résidu acide aminé en position, par exemple, 41 dans la séquence d'un variant de la protéine TAT est en fait un résidu acide aminé qui correspond après alignement à l'acide aminé en position 41 dans la séquence de la protéine TAT native du virus VIH1.

De manière plus particulière, un variant de la protéine TAT selon l'invention présente notamment un phénotype trans-dominant caractérisé par une activité trans-activatrice inférieure à environ 50% par rapport à celle de la protéine TAT native, de manière avantageuse inférieure à 20% et de préférence inférieure à 10% et une activité inhibitrice de la fonction TAT native, telle que déterminée dans un rapport de concentration variant/TAT native de 10/1, supérieure à 50%, de manière avantageuse supérieure à 75% et de préférence supérieure à 90%. Différentes méthodes de mesure de l'activité trans-activatrice sont actuellement connues. Elles varient selon le gène rapporteur utilisé. A titre d'exemple on cite la méthode mettant en oeuvre le gène CAT placé sous le contrôle du LTR5' du virus VIH, décrite dans les exemples ci-après.

Un variant de la protéine TAT selon l'invention peut être dérivé d'un fragment fonctionnel de ladite protéine et être muté au niveau d'un résidu comme indiqué ci-avant, la numérotation des acides aminés étant également alignée avec celle établie pour la protéine TAT native du virus VIH1 de référence. Par fragment fonctionnel de la protéine TAT, on entend tout fragment de ladite protéine qui est capable d'induire une trans-activation de l'expression des gènes placés sous le contrôle d'un promoteur comportant la séquence TAR, tel que le promoteur du virus VIH. Par exemple, un variant de la protéine TAT selon l'invention peut être généré après mutation d'un fragment de la protéine TAT, tel qu'un fragment débutant à l'acide aminé 1 et s'arrêtant à l'acide aminé 72.

Un variant de la protéine TAT selon l'invention peut comporter plusieurs mutations par rapport à la protéine TAT native du VIH. Il peut comporter une combinaison d'au moins 2 mutations telles que décrites ci-avant.

De manière plus particulière, un variant de la protéine TAT selon l'invention a notamment une séquence dont le degré d'homologie avec la séquence TAT native du VIH1 est supérieur à 75%, de manière avantageuse supérieur à 90% et de préférence supérieur à 95%.

Bien entendu, les résidus à muter peuvent être remplacés par tout acide aminé autre que le résidu naturel, sauf le résidu en position 41 qui peut être remplacé par une arginine, asparagine, acide aspartique, cystéine, glycine, acide glutamique, glutamine, histidine, isoleucine, leucine, méthionine, phénylalanine, proline, serine, tryptophane, tyrosine et valine ; et le résidu en position 47 qui peut être remplacé par une arginine, asparagine, acide aspartique, cystéine, glycine, acide glutamique, glutamine, isoleucine, leucine, lysine, méthionine, phénylalanine, proline, serine, thréonine, tryptophane et valine.

Selon un aspect particulièrement avantageux de l'invention, un variant TAT comprenant en position 38 un acide aminé ayant une chaîne latérale acide tel qu'un acide glutamique, un acide aspartique ou de préférence, un acide aspartique.

L'invention concerne également un fragment d'ADN codant pour ledit variant de la protéine TAT. La séquence codant pour un variant de la protéine TAT peut être obtenue selon les techniques classiques du génie génétique, par exemple par mutagénèse dirigée. Ainsi le fragment d'ADN comprenant le gène codant pour la protéine TAT native du VIH ou une portion de ladite séquence est cloné dans un phage de type M13. La séquence est modifiée en mettant en oeuvre un oligonucléotide approprié, de manière à obtenir la ou les mutation(s) désirée(s).

L'invention inclut également une cassette d'expression comprenant un fragment d'ADN codant pour un variant de la protéine TAT selon l'invention, placé sous le contrôle d'éléments appropriés permettant son expression. Une cassette d'expression est notamment utile pour permettre de synthétiser un variant de la protéine TAT dans un système d'expression hétérologue ou bien comme élément d'un vecteur destiné à la thérapie génique tel qu'un vecteur rétroviral ou adénoviral. Par éléments appropriés permettant l'expression du fragment d'ADN codant pour un variant de la protéine TAT, on entend l'ensemble des éléments permettant la transcription dudit fragment d'ADN en ARNm et la traduction de l'ARNm en protéine.

Ces éléments comportent notamment un promoteur approprié. Dans le contexte de l'invention, le terme "promoteur" signifie tout élément de contrôle transcriptionnel impliqué dans l'expression d'un fragment d'ADN en fonction de la cellule hôte qui a été retenue. De tels éléments de contrôle sont bien connus de l'homme de l'art et sont insérés dans la cassette d'expression par les techniques conventionnelles du génie génétique.

Le promoteur retenu peut être d'origine cellulaire ou virale. Le promoteur peut être de type ubiquitaire permettant une expression permanente du fragment d'ADN. Tel est le cas du promoteur PGK (phosphoglycerate-kinase) fonctionnel chez la levure ou du promoteur tardif du virus SV40 (Simian Virus 40), du promoteur du gène HMG (Hydroxy-Methyl-Glutaryl coenzyme A réductase), du promoteur du gène de la thymidine kinase (TK) du virus Herpes Simplex de type 1 (HSV1), du promoteur EIII et MLP (Major Late Promoter) de l'adénovirus et du LTR du virus MoMuLV (Moloney Murine Leukemia Virus) fonctionnels dans les cellules animales.

Le terme "promoteur" inclut également un promoteur de type régulable, par exemple un promoteur tissu-spécifique comme le promoteur des gènes d'immunoglobulines spécifique des cellules lymphocytaires.

En outre, la cassette d'expression peut contenir :
(1) des UAS (Upstream Activating Sequence) tels les UAS du gène GAL4 (Galactose) de levure,
(2) des enhancers qui peuvent être placés soit en amont du promoteur ou en aval du fragment d'ADN et qui permettent d'augmenter les niveaux d'expression, tel l'enhancer du gène CD2 humain. De plus, ce dernier comprend des éléments permettant de cibler sélectivement l'expression du fragment d'ADN dans les cellules lymphocytaires de type T,
(3) des séquences introniques connues pour améliorer l'expression génique chez les eucaryotes supérieurs, tel l'intron du virus SV40,
(4) des signaux d'épissage comme par exemple ceux du virus SV40 permettant l'élimination des séquences introniques dans les ARNm destinés à être traduits en protéines,
(5) les éléments impliqués dans la terminaison de la transcription, tels les signaux de polyadenylation du virus SV40.

D'une manière générale, la cassette d'expression peut permettre l'expression d'un variant de la protéine TAT à l'intérieur d'une cellule hôte, et de préférence à l'intérieur d'un lymphocyte.

La cassette d'expression peut aussi permettre la production du variant de la protéine TAT dans le milieu de culture d'où il peut être facilement récolté. Dans ce cas, le fragment d'ADN code pour un précurseur du variant de la protéine TAT comprenant, en amont de la séquence mature, un peptide signal permettant la sécrétion dudit variant de la cellule hôte. De tels peptides signal sont bien connus de l'homme de l'art, par exemple la séquence signal du facteur Mat alpha de la levure.

La cassette d'expression est insérée dans un vecteur d'expression qui sert à transformer une cellule hôte dans laquelle le promoteur et les éléments appropriés sont fonctionnels. Un tel vecteur peut être sous la forme d'un plasmide ou d'un vecteur viral, par exemple un vecteur rétroviral dérivé du MoMuLV ou un vecteur adénoviral dérivé de l'adénovirus de type 5.

La présente invention s'étend aux cellules comprenant une cassette d'expression selon l'invention, les cellules pouvant être d'origine eucaryote ou procaryote. La cellule hôte peut être une bactérie, un champignon, par exemple une levure ou une cellule de mammifère. De préférence, la cellule hôte sera une cellule humaine de la lignée hématopoïétique.

L'invention concerne également un procédé de préparation d'un variant de la protéine TAT selon l'invention, selon lequel:
(1) on cultive une cellule eucaryote ou procaryote selon l'invention, comportant un fragment d'ADN codant pour ledit variant, dans un milieu de culture approprié et,
(2) on récolte le variant à partir du milieu de culture ou de ladite cellule.

L'invention couvre également un variant de la protéine TAT obtenu par la mise en oeuvre dudit procédé.

L'invention s'étend également à l'utilisation thérapeutique d'un variant de la protéine TAT selon l'invention d'une cassette d'expression ou d'une cellule produisant ledit variant pour inhiber la réplication du virus VIH. De manière préférée, la présente invention concerne l'utilisation d'un variant de la protéine TAT ou d'une cassette d'expression ou d'une cellule produisant ledit variant pour la fabrication d'un médicament destiné au traitement ou à la prévention du SIDA chez les mammifères, de préférence les humains.

De manière avantageuse, une cassette d'expression ou une cellule selon l'invention, peuvent être utilisées à des fins de thérapie génique anti-SIDA. La cassette d'expression est insérée dans un vecteur de type rétroviral qui est introduit dans les cellules souches de la lignée hématopoïétique prélevées de la moelle osseuse d'un individu (de préférence infecté par le VIH). Les cellules souches ainsi transfectées sont réinjectées au donneur. Elles sont capables de se diviser et de se différencier entre autre en lymphocytes. Les lymphocytes qui comprennent le vecteur rétroviral vont exprimer de façon prolongé dans le temps le gène codant pour un variant trans-dominant de la protéine TAT rendant lesdites cellules résistantes à l'infection par le VIH.

L'invention a également trait à une composition pharmaceutique qui comprend à titre d'agent thérapeutique ou prophylactique un variant selon l'invention, une cassette d'expression ou une cellule produisant un tel variant. Une composition pharmaceutique selon l'invention peut être préparée selon les techniques communément en usage. Par exemple, on associe à l'agent thérapeutique ou prophylactique en quantité thérapeutiquement efficace, un support, un diluant ou un adjuvant acceptable.

Enfin, l'invention concerne une méthode de traitement selon laquelle on administre une quantité thérapeutiquement efficace d'un variant de la protéine TAT selon l'invention, une cassette d'expression ou une cellule produisant un tel variant à un patient.

L'invention est illustrée ci-après par référence aux figures suivantes :
La Figure 1 est une représentation schématique du génome du virus VIH, les gènes codant pour les protéines étant représentés selon le cadre de lecture utilisé ; LTR : boîtes noires ; gènes codant pour les protéines de structure : boîtes grisées ; gènes codant pour les protéines de régulation : boîtes blanches.
La Figure 2 est une représentation schématique des différents domaines constituant la protéine TAT native du virus VIH, les acides aminés constituant chaque domaine étant indiqués au dessus de chacun des domaines.

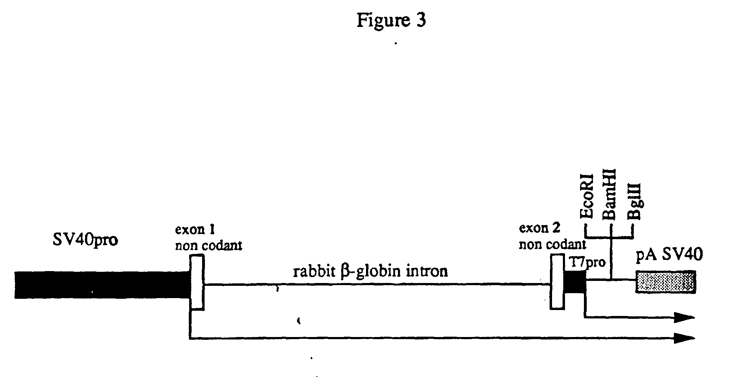

La Figure 3 est une représentation schématique du vecteur d'expression eucaryote pSG5 qui comprend en séquence : le promoteur SV40 (SV40 pro ; boite noire), le fragment 3' de l'exon 1 non codant du gène de la β globine de lapin (boîte blanche), l'intron du gène de la β globine de lapin (trait continu), le début non codant de l'exon 2 du gène de la β globine de lapin (boîte blanche), le promoteur du bactériophage T7 (T7 pro ; boîte noire), des sites uniques EcoRI, BamHI, BglII permettant le clonage d'un gène d'intérêt et le signal de polyadénylation du virus SV40 (pA SV40 ; boîte grisée).

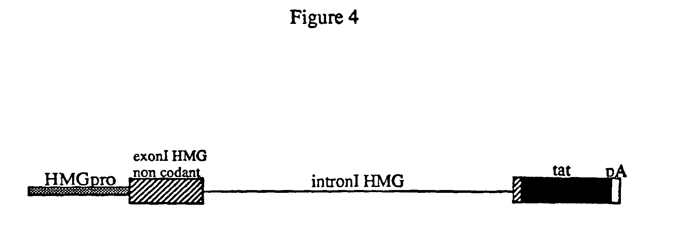

La Figure 4 est une représentation schématique du vecteur d'expression de la protéine TAT native pHMG-Tat qui comprend en séquence : le promoteur du gène HMG (HMGpro), l'exon I non codant du gène HMG (boîte rayée), l'intron I et le site accepteur pour l'épissage du gène HMG, l'ADN-copie codant pour les 86 acides aminés de la protéine TAT (boîte noire) et le signal de polyadenylation de SV40 (pA, boîte blanche).

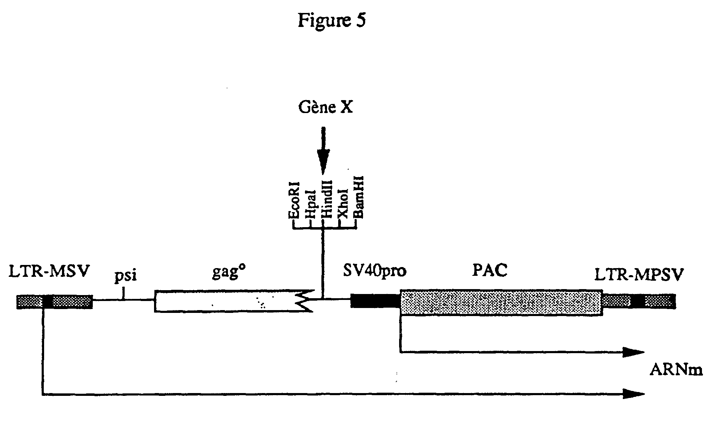

La Figure 5 est une représentation schématique du vecteur rétroviral pLXSP comprenant en séquence le LTR 5' du virus MSV (Murine Sarcoma virus) incluant la région promotrice virale, une région d'encapsidation (psi), la partie 5' du gène gag viral (gag°), des sites multiples de clonage permettant l'insertion de gènes hétérologues (gène x), le promoteur du virus SV40 dirigeant l'expression du gène de résistance à la puromycine (PAC) et le LTR3' du virus MPSV (Myelo Proliferative Sarcoma Virus) incluant les signaux de polyadénylation.

### EXEMPLES

### EXEMPLE 1 : construction du plasmide d'expression pTG2332, expression du variant TAT(Phe³⁸ -> Asp) et caractérisation du phénotype trans-dominant.

Un fragment d'ADN comportant l'ADN-copie correspondant aux 2 exons du gène *tat* du génome VIH-isolat Lai (Wain-Hobson et al., Cell, 1985, **40**, 9-17 ; Sodroski et al., Science, 1985, **229**, 74-77) a été modifié afin de créer un site BamHI en 5' de l'ATG initiateur. En outre, un site BamHI existe naturellement 53pb après le codon stop du gène *tat.* Le fragment BamHI de 300pb comportant le gène *tat* non muté a été purifié par le kit Gene Clean (Bio 101, Inc, P.O. Box 2284, La Jolla) et inséré dans un vecteur M13TG130 (Kieny et al., Gene, 1983, **26**, 91-99) afin de donner le vecteur M13TG2306, sur lequel sont effectuées les mutagénèses.

La mutation du codon codant pour le résidu 38 est sur M13TG2306 en utilisant le kit Amersham (Oligonucleotide-directed in vitro mutagenesis system, version 2.1 RPN 1523) et en mettant en oeuvre l'oligonucléotide décrit dans l'identificateur de séquence SEQ ID NO:1.

L'oligonucléotide a été conçu de manière à remplacer le codon codant pour le résidu phénylalanine (Phe) en position 38 par un acide aspartique (Asp) et à créer également un site de restriction HindIII au niveau du codon codant pour le résidu en position 42 de la séquence TAT sans modifier l'acide aminé pour lequel il code. La présence d'un site de restriction permet d'identifier facilement les vecteurs mutés des parentaux lors de l'analyse des ADNs du bactériophage par la technique de mini-préparation décrite par Maniatis et al. (Molecular cloning : a laboratory manual, 1989, Cold Spring Harbor Laboratory).

Après mutagénèse, le fragment BamHI comportant le gène *tat* muté est alors transféré dans le vecteur d'expression eucaryote pSG5 (Green et al., Nucleic Acids Res, 1988, **16**, 369) illustré dans la Figure 3. Le plasmide en résultant, pTG2332, permet l'expression du variant TAT (Phe³⁸ -> Asp).

Les variants trans-dominants TAT de l'art antérieur, respectivement le variant TAT (Lys⁴¹ -> Ala) et ΔTAT (Gln⁵⁴ -> stop) ont été créés par mutagénèse dirigée du vecteur M13TG2306, en mettant en oeuvre les oligonucléotides respectivement décrits dans la SEQ ID NO: 2 permettant de modifier le codon codant pour le résidu lysine en position 41 par une alanine et, dans la SEQ ID NO:3 permettant de créer un codon stop à la place du codon codant pour le résidu glutamine en position 54.

Après mutagénèse, le gène *tat* muté est inséré dans le vecteur d'expression eucaryotique pSG5, donnant respectivement pTG2351 permettant la production du mutant TAT (Lys⁴¹ -> Ala) et pTG2352 permettant la production de ΔTAT. Ces mutants de l'art antérieur sont utilisés comme témoin positif de trans-dominance. Leur activité trans-activatrice ainsi que leur capacité à inhiber la protéine TAT native, est évaluée selon des conditions strictement identiques à celles utilisées pour les variants TAT selon l'invention et qui sont décrites ci-après.

On évalue l'activité trans-activatrice du variant TAT (Phe³⁸ -> Asp) par transfection transitoire en cellules HeLa du vecteur d'expression pTG2332 avec le vecteur rapporteur LTR-CAT (Emerman et al., EMBO J., 1987, **6**, 37-55). Les cellules sont transfectées selon la technique au phosphate de calcium (Maniatis et al., Molecular cloning : a laboratory manual, 1989, Cold Spring Harbor Laboratory). D'autres protocoles permettant d'introduire un acide nucléique dans une cellule peuvent également être employés, tels que la technique au sulfate de dextrane, l'électroélution, les méthodes basées sur les chocs osmotiques ou la microinjection d'une cellule sélectionnée.

On cultive les cellules humaines HeLa à 37°C en présence de 5% de CO₂ dans un milieu MEM (Eagle's minimum essential medium) complémenté avec 10% de sérum foetal de veau, 1% d'acides aminés non essentiels, 1% de glutamine et 1% de gentamycine.

On purifie les ADNs plasmidiques sur gradient de chlorure de césium. On co-transfecte 1µg de pTG2332 et 0,5µg du plasmide LTR-CAT dans les cellules HeLa en culture, étalées à raison de 4x10⁵ cellules par boîte. La transfection de 1µg de pHMG-Tat (Figure 4) et 0,5µg de LTR-CAT constitue le témoin positif de transactivation. Le vecteur pHMG-tat est issu du clonage du fragment BamHI comprenant l'ADN-copie du gène *tat* codant pour les 86 acides aminés de la protéine TAT, dans le site BamHI du vecteur pHMG (Mehtali et al., Gene, 1990, **91**, 179-184). Les ADNs plasmidiques devant être transfectés, sont repris dans 420µl de TE (Tris-HCl 10mM, pH 7,5 ; EDTA 1mM) et mélangés à 60µl de CaCl₂ 2M. On ajoute le mélange à 480µl d'HBSx2 (NaCl 280mM ; HEPES 50mM ; Na₂HPO₄-2H₂O 1,5mM ajusté à pH 7,12 avec NaOH) en agitant légèrement le tube. Après 15mn, le précipité est versé goutte à goutte sur les cellules. On change le milieu 24h après la transfection afin d'éliminer l'excès de précipité. On analyse les extraits cellulaires 48h après les transformations afin d'évaluer l'expression du gène CAT.

On recueille les cellules HeLa par grattage dans un tampon CAT (Tris-HCl 150mM pH8 ; KCl 60mM ; NaCl 15mM ; EDTA 2mM ; spermine 0,15mM ; dithiothréitol (DTT) 1mM ; phénylméthylsulfonyl fluoride (PMSF) 0,4mM). On réalise 3 cycles de congélation-décongélation dans l'azote liquide suivi d'une incubation à 37°C pendant 4mn. On incube le lysat ainsi obtenu à 65°C pendant 10mn et on centrifuge à 10000rpm pendant 10mn de façon à éliminer les débris cellulaires. On récupère le surnageant sur lequel on détermine la concentration protéique par test colorimétrique Biorad.

On incube 15µl d'extrait cellulaire éventuellement dilué et complété à 800µl avec H₂O et 200µl de réactif Biorad dans H₂O à température ambiante pendant 10 à 60mn. On détermine la concentration protéique par mesure de la densité optique à 595nm par rapport à une gamme étalon de sérum albumine bovine.

On détermine l'expression du gène CAT sur un aliquote d'extrait correspondant à 10 à 20µg en protéines. On incube le volume adéquat d'extrait cellulaire préalablement complété à 300µl avec du tampon Tris-HCl 0,25 M pH 7,8 avec 32µl d'acétyl-coenzyme A 5mM et 20µl (0,5µCi) de ¹⁴C chloramphénicol à 37°C pendant 1h30. Après extraction à l'acétate d'éthyle et centrifugation à 10000rpm pendant 5mn, on lyophylise la phase supérieure. Celle-ci est reprise dans 15µl d'acétate d'éthyle, déposée sur une plaque de gel de silice 60F₂₃₄ (Merck) et analysée par chromatographie en couche mince (migration dans un tampon chloroforme/méthanol 19/1 pendant 1h30). La chromatographie est révélée par autoradiographie et les bandes correspondantes au chloramphénicol acétylé sont découpées. Le taux de radioactivité est évalué par comptage en scintillation. Le pourcentage d'activité trans-activatrice du mutant est calculé par rapport au taux mesuré avec le témoin positif (pHMG-Tat + LTR-CAT), celui-ci représentant 100% d'activité trans-activatrice.

On évalue l'activité trans-dominante du variant TAT (Phe³⁸ -> Asp) en mesurant sa capacité à inhiber la fonction trans-activatrice de la protéine TAT native. L'activité trans-dominante est déterminée par transfection transitoire en cellules HeLa du vecteur d'expression pTG2332 avec le vecteur rapporteur LTR-CAT et le vecteur d'expression de la protéine TAT native pHMG-Tat. On co-transfecte 10µg de pTG2332, 1µg de pHMG-Tat et 0,5µg de LTR-CAT selon le protocole décrit précédemment.

48h après la transfection, les extraits cellulaires sont préparés et leur concentration en protéines déterminée. Un aliquote d'extrait correspondant à 10 à 20µg en protéines est soumis à l'essai CAT. Les conditions techniques mises en oeuvre ont été décrites en détail ci-avant. Le pourcentage d'inhibition de la protéine TAT native par le variant TAT (Phe³⁸ - > Asp) est calculé par rapport au témoin positif d'activation résultant de la co-transfection de pHMG-Tat et de LTR-CAT qui représente 0%. Les résultats obtenus sont reportés dans le tableau 1.

| Vecteur d' expression | Modification de l'acide aminé | TA⁽¹⁾ | TD⁽²⁾ |
|---|---|---|---|
| pTG 2332 | Phe - > Asp en position 38 | 1,4 % | 98,0 % |
| pTG 2333 | Thr - > Ala en position 40 | 15,6 % | 83,0 % |
| pTG 2340 | Lys -> Glu en position 41 | 3,0 % | 97,0 % |
| pTG 2358 | Ile - > Ser en position 45 | 47,0 % | 62,5 % |
| pTG 2348 | Tyr - > Arg en position 47 | 56,0 % | 78,0 % |
| pTG 2351 | Lys - > Ala en position 41 | 172,0 % | 0,0 % |
| pTG 2352 | Gln - > Stop en position 54 | 28,0 % | 92,0 % |
| pHMG Tat | Protéine TAT native | 100,0 % | 0,0 % |

| | | | |
|---|---|---|---|
| (1): Activité trans-activatrice. | | | |
| (2): Activité inhibitrice de la protéine TAT native ou activité trans-dominante. | | | |

Le variant TAT (Phe³⁸-> Asp) est un mutant trans-dominant efficace puisque l'activité trans-activatrice qu'il présente est infime (expression du gène CAT évaluée à 1,4% par rapport à celle mesurée avec la protéine TAT native) et puisqu'il inhibe fortement l'activité de la protéine TAT native (98% d'inhibition en présence de pTG2332).

On peut constater que le variant TAT (Lys⁴¹ -> Ala) décrit par Green et al. et qui posséderait un phénotype trans-dominant d'après les auteurs, donne des résultats tout à fait contradictoires dans ces expériences. En effet, il présente une activité trans-activatrice particulièrement élevée (172%) et semble donc agir en coopération avec la protéine TAT native. En outre, ce variant n'exerce aucun effet dominant sur la fonction TAT native (0% d'activité trans-dominante).

Le variant de l'art antérieur ΔTAT produit par pTG2352 constitue bien un témoin de trans-dominance, ce qui confirme la validité de ces expériences. Il inhibe l'activité trans-activatrice de la protéine TAT native à 92%. Cependant, ce variant est capable d'induire une expression du gène CAT placé sous le contrôle du LTR du VIH (28%), la trans-activation étant toutefois inférieure à celle mesurée avec le témoin positif pHMG-Tat.

### EXEMPLE 2 : construction du vecteur d'expression pTG2333, expression du variant TAT (Thr⁴⁰ - > Ala) et caractérisation du phénotype de trans-dominance.

Le vecteur M13TG2306 est soumis à une mutagénèse dirigée afin de remplacer le codon codant pour le résidu thréonine (Thr) en position 40 de la protéine TAT du VIH par un codon codant pour un résidu alanine (Ala) et d'introduire des mutations silencieuses de façon à créer un site de restriction HindIII au niveau du codon codant pour le résidu 42 permettant une identification rapide des vecteurs mutés. L'oligonucléotide utilisé est le décrit dans la SEQ ID NO:4. Le gène *tat* muté est transféré dans le vecteur pSG5 donnant lieu à pTG2333 qui permet de produire le variant TAT (Thr⁴⁰ -> Ala).

Le vecteur pTG2333 est co-transfecté transitoirement dans les cellules HeLa avec le vecteur LTR-CAT afin de déterminer l'activité trans-activatrice du variant TAT (Thr⁴⁰ -> Ala) selon le protocole décrit précédemment. Parallèlement, son activité trans-dominante est déterminée par co-transfection de pTG2333, pHMG-Tat et LTR-CAT dans un rapport de concentration de 10:1:0,5.

Les résultats sont présentés dans le tableau 1. On peut constater que le variant TAT (Thr⁴⁰ -> Ala) présente un phénotype trans-dominant tel que défini selon l'invention. Il inhibe la fonction de la protéine TAT native à 83% et il conserve une faible activité trans-activatrice de 15,6%

### EXEMPLE 3 : construction du vecteur d'expression pTG2340, expression du variant TAT (Lys⁴¹-> Glu) et caractérisation du phénotype de trans-dominance.

Le vecteur M13TG2306 est soumis à une mutagénèse dirigée afin de remplacer le codon codant pour le résidu lysine (Lys) en position 41 de la protéine TAT du VIH par un codon codant pour un résidu acide glutamique (Glu) et d'introduire des mutations silencieuses de façon à créer un site de restriction HindIII au niveau du codon codant pour le résidu 42 permettant une identification rapide des vecteurs mutés. L'oligonucléotide utilisé est décrit dans la SEQ ID NO:5. Le gène *tat* muté est transféré dans le vecteur pSG5 donnant lieu à pTG2340 qui permet de produire le variant TAT (Lys⁴¹ -> Glu).

Le vecteur pTG2340 est co-transfecté transitoirement dans les cellules HeLa avec le vecteur LTR-CAT afin de déterminer l'activité trans-activatrice du variant TAT (Lys⁴¹ -> Glu) selon le protocole décrit précédemment. Parallèlement, son activité trans-dominante est déterminée par co-transfection de pTG2340, pHMG-Tat et LTR-CAT dans un rapport de concentration de 10:1:0,5.

Les résultats sont présentés dans le tableau 1. On peut constater que le variant TAT (Lys⁴¹ - > Glu) présente un phénotype trans-dominant tel que défini selon l'invention. L'activité trans-activatrice du variant est faible (3% de l'activité conférée par la protéine TAT native) et il est capable d'inhiber efficacement la fonction trans-activatrice de la protéine TAT native (97% d'inhibition).

On peut noter que le variant TAT (Lys⁴¹ -> Glu) produit par pTG2340 et le variant de l'art antérieur TAT (Lys⁴¹ -> Ala) qui se comporte comme la protéine TAT native, sont mutés sur le même résidu. Selon la modification réalisée, le phénotype du variant obtenu peut donc être totalement différent. Ainsi, le remplacement de la Lys⁴¹ par un Glu, tel que divulgué par la demanderesse, confère au variant généré un phénotype trans-dominant.

### EXEMPLE 4 : construction du vecteur d'expression pTG2358, expression du variant TAT (Ile⁴⁵-> Ser) et caractérisation du phénotype de trans-dominance.

Le vecteur M13TG2306 est soumis à une mutagénèse dirigée afin de remplacer le codon codant pour le résidu isoleucine (Ile) en position 45 de la protéine TAT du VIH par un codon codant pour un résidu serine (Ser) et de créer un site de restriction BamHI au niveau du codon codant pour le résidu 45 permettant une identification rapide des vecteurs mutés. L'oligonucléotide utilisé est décrit dans la SEQ ID NO:6. Le gène *tat* muté est transféré dans le vecteur pSG5 donnant lieu à pTG2358 qui permet de produire le variant TAT (Ile⁴⁵ -> Ser).

Le vecteur pTG2358 est co-transfecté transitoirement dans les cellules HeLa avec le vecteur LTR-CAT afin de déterminer l'activité trans-activatrice du variant TAT (Ile⁴⁵ -> Ser) selon le protocole décrit précédemment. Parallèlement, son activité trans-dominante est déterminée par co-transfection de pTG2358, pHMG-Tat et LTR-CAT dans un rapport de concentration de 10:1:0,5.

Les résultats sont présentés dans le tableau 1. On peut constater que le variant TAT (Ile⁴⁵ -> Ser) présente un phénotype trans-dominant tel que défini selon l'invention. Il inhibe partiellement la fonction de la protéine TAT native à 62,5% et il conserve une activité trans-activatrice modérée de 47%

### EXEMPLE 5 : construction du vecteur d'expression pTG2348, expression du variant TAT (Tyr⁴⁷ - > Arg) et caractérisation du phénotype de trans-dominance,

Le vecteur M13TG2306 est soumis à une mutagénèse dirigée afin de remplacer le codon codant pour le résidu tyrosine (Tyr) en position 47 de la protéine TAT du VIH par un codon codant pour un résidu arginine (Arg) et créer un site de restriction XbaI au niveau du codon codant pour le résidu 47 permettant une identification rapide des vecteurs mutés. L'oligonucléotide utilisé est décrit dans la SEQ ID NO:7. Le gène *tat* muté est transféré dans le vecteur pSG5 donnant lieu à pTG2348 qui permet de produire le variant TAT (Tyr⁴⁷ -> Arg).

Le vecteur pTG2348 est co-transfecté transitoirement dans les cellules HeLa avec le vecteur LTR-CAT afin de déterminer l'activité trans-activatrice du variant TAT (Tyr⁴⁷ -> Arg) selon le protocole décrit précédemment. Parallèlement, son activité trans-dominante est déterminée par co-transfection de pTG2348, pHMG-Tat et LTR-CAT dans un rapport de concentration de 10:1:0,5.

Les résultats sont présentés dans le tableau 1. On peut constater que le variant TAT (Tyr⁴⁷ -> Arg) présente un phénotype trans-dominant modéré tel que défini selon l'invention. Il inhibe la fonction de la protéine TAT native à 78% et il conserve une activité trans-activatrice de 56%

### EXEMPLE 6 : Etablissement de lignées cellulaires stables exprimant un variant trans-dominant TAT et évaluation de leur résistance à l'infection par le virus VIH.

Des lignées cellulaires stables produisant le variant trans-dominant TAT (Phe³⁸ - > Asp) ont été établies afin de valider l'efficacité des variants pour une éventuelle utilisation in vivo, par exemple dans un protocole de thérapie génique. Pour évaluer la résistance des cellules à une infection par le VIH, il est nécessaire que celles-ci soient infectables par le virus, c'est à dire qu'elles expriment le récepteur CD₄ humain à leur surface.

Deux types de lignées cellulaires ont été établies :
- Les cellules CEM-A3 (ATCC CCL119) dérivées des cellules T humaines, possèdent le récepteur CD₄ et sont donc naturellement infectables par le VIH.
- Les cellules HeLa (ATCC CCL2) qui sont co-transfectées avec un vecteur d'expression ubiquitaire du récepteur CD₄ humain pTG620 afin de leur conférer une susceptibilité à l'infection par le VIH. Le vecteur pTG620 provient de l'introduction dans le site EcoRV du vecteur pHMG d'un fragment EcoRI traité par le fragment klenow de l'ADN polymerase d'E.coli comprenant l'ADN-copie du récepteur CD4 humain (Maddon et al., Cell, 1986, **47**, 333-348).

Le fragment Bam HI comportant le gène codant pour le variant trans-dominant TAT (Phe³⁸ -> Asp) est inséré dans le site BamHI du vecteur rétroviral pLXSP (Figure 5) générant pTG2350. Le vecteur pLXSP dérive du pLXSN (Miller et Rossman, Biotechniques, **7**, 1989, 980-988) et provient du remplacement du gène néomycine par un gène conférant une résistance à la puromycine et du LTR3' du MSV par celui du MPSV.

L'ADN plasmidique de pTG2350 est introduit dans les cellules CEM-A3 par électroporation. On reprend 20µg d'ADN purifié sur gradient de chlorure de césium dans 20µl de tampon PBS (KH₂ PO₄ 1mM ; KCl 2mM ; NaCl 136mM, Na₂ HPO₄ 3mM). On mélange la préparation d'ADN à 2 X 10⁷ cellules resuspendues dans 180µl de PBS dans une cuve à électroporation (Biorad). On préincube à température ambiante pendant 10 minutes. Après l'électroporation (Gene pulser Biorad, voltage 210V, capacitance 960µF), on maintient les cellules à température ambiante pendant 10 minutes et on les incube à 37°C dans 6 ml de RPMI (Gibco-BRL) en présence de 5% de CO₂. On isole les clones CEM-A3 résistants à la puromycine par dilution limite (puromycine 0,5µg/ml).

L'ADN plasmidique pTG2350 est co-transfecté dans les cellules HeLa avec le pTG620 par la technique de transfection au phosphate de calcium précédemment décrite. On isole les clones de cellules HeLa résistants à la puromycine par repiquages successifs (puromycine 2µg/ml). On analyse l'expression du récepteur CD₄ humain à la surface des cellules par fluorocytométrie (FACS : Fluorescence Activated Cell Sorter, scan Becton Dickinson) à l'aide de l'anticorps Leu3A marqué à la phycoérythrine (Becton Dickinson) dirigée contre ce récepteur.

On détecte l'expression de la protéine TAT dans la lignée cellulaire CEM-A3 en réalisant des transfections transitoires des vecteurs LTR-CAT et pHMG-Tat selon la technique décrite précédemment.

Trois clones issus de la lignée cellulaire CEM-A3 résistants à la puromycine et exprimant le variant TAT trans-dominant et pour lequel différents degrés d'inhibition de la protéine TAT native ont été observés, sont infectés par le virus VIH. La propagation du virus peut être évaluée par mesure de l'activité reverse-transcriptase dans les surnageants de culture. On a ainsi pu détecter un décalage de 2 jours de la multiplication virale dans un des clones de la lignée cellulaire CEM-A3 exprimant le variant trans-dominant par rapport au résultat obtenu dans les cellules CEM-A3 n'exprimant pas un variant trans-dominant. De plus, l'activité reverse-transcriptase est réduite de 90% dans ledit clone.

## Revendications

1. Un variant trans-dominant de la protéine TAT du virus VIH ou d'un fragment fonctionnel de ladite protéine capable d'induire une transactivation de l'expression d'un ou plusieurs gène(s) placé(s) sous le contrôle d'un promoteur comportant la séquence TAR, **caractérisé en ce que** ledit variant comprend une mutation sélectionnée parmi les mutations consistant en la présence en position 38 d'un résidu ayant une chaîne latérale acide tel qu'un acide glutamique ou un acide aspartique.

2. Variant selon la revendication 1, **caractérisé en ce que** ledit variant est dérivé de la protéine TAT de VIH 1.

3. Variant selon les revendications 1 à 2 comprenant un acide aspartique en position 38.

4. Fragment d'ADN codant pour un variant de la protéine TAT selon l'une des revendications 1 à 3.

5. Cassette d'expression comprenant un fragment d'ADN selon la revendication 4, placé sous le contrôle d'éléments appropriés permettant son expression.

6. Cellule eucaryote ou procaryote comprenant une cassette d'expression selon la revendication 5.

7. Procédé de préparation d'un variant selon l'une des revendications 1 à 3, selon lequel :
- on cultive une cellule eucaryote ou procaryote selon la revendication 6 dans un milieu de culture approprié et,
- on récolte le variant produit à partir du milieu de culture ou de ladite cellule.

8. Variant de la protéine TAT obtenu par la mise en oeuvre d'un procédé selon la revendication 7.

9. Variant selon l'une des revendications 1 à 3, cassette d'expression selon la revendication 5, ou cellule selon la revendication 6, pour leur utilisation à titre de médicament.

10. Composition pharmaceutique destinée au traitement ou à la prévention d'une infection à VIH qui comprend à titre d'agent thérapeutique ou prophylactique un variant selon l'une des revendications 1 à 3, une cassette d'expression selon la revendication 5 ou une cellule selon la revendication 6.

11. Utilisation d'un variant selon l'une des revendications 1 à 3, d'une cassette d'expression selon la revendication 5 ou d'une cellule selon la revendication 6, pour l'obtention d'un médicarnent destiné à inhiber la réplication du virus VIH.

## Patentansprüche

1. Trans-dominante Variante des TAT-Proteins des HIV-Virus oder von einem funktionalen Fragment dieses Proteins, welche in der Lage ist, eine Transaktivierung der Expression von einem oder mehreren Gen(en), welches bzw. welche unter die Kontrolle eines die TAR-Sequenz umfassenden Promotors gestellt ist bzw. sind, zu induzieren, **dadurch gekennzeichnet, dass** die Variante eine Mutation umfasst, welche ausgewählt wird unter den Mutationen, welche aus dem Vorhandensein eines Rests mit einer sauren Seitenkette, wie einer Glutaminsäure oder einer Asparaginsäure, an Position 38 bestehen.

2. Variante nach Anspruch 1, **dadurch gekennzeichnet, dass** die Variante von dem TAT-Protein von HIV1 abgeleitet ist.

3. Variante nach den Ansprüchen 1 bis 2, welche eine Asparaginsäure an Position 38 umfasst.

4. DNA-Fragment, welches eine Variante des TAT-Proteins nach einem der Ansprüche 1 bis 3 kodiert.

5. Expressionskassette, welche ein DNA-Fragment nach Anspruch 4, welches unter die Kontrolle von geeigneten Elementen, welche dessen Expression erlauben, gestellt ist, umfasst.

6. Eukaryotische oder prokaryotische Zelle, welche eine Expressionskassette nach Anspruch 5 umfasst.

7. Verfahren zur Herstellung einer Variante nach einem der Ansprüche 1 bis 3, gemäß welchem:
- man eine eukaryotische oder prokaryotische Zelle nach Anspruch 6 in einem geeigneten Kulturmedium kultiviert und
- man die produzierte Variante ausgehend von dem Kulturmedium oder der Zelle
gewinnt.

8. Variante des TAT-Proteins, welche durch das Ausführen eines Verfahrens nach Anspruch 7 erhalten wird.

9. Variante nach einem der Ansprüche 1 bis 3, Expressionskassette nach Anspruch 5 oder Zelle nach Anspruch 6 für deren Verwendung als Arzneimittel.

10. Pharmazeutische Zusammensetzung, welche für die Behandlung oder die Verhütung einer HIV-Infektion bestimmt ist, welche als therapeutisches oder prophylaktisches Mittel eine Variante nach einem der Ansprüche 1 bis 3, eine Expressionskassette nach Anspruch 5 oder eine Zelle nach Anspruch 6 umfasst.

11. Verwendung einer Variante nach einem der Ansprüche 1 bis 3, einer Expressionskassette nach Anspruch 5 oder einer Zelle nach Anspruch 6 für die Herstellung eines Arzneimittels, welches dazu bestimmt ist, die Replikation des HIV-Virus zu hemmen.

## Claims

1. Transdominant variant of the HIV virus TAT protein or of a functional fragment of said protein which is capable of inducing transactivation of the expression of one or more genes placed under the control of a promoter containing the TAR sequence, **characterized in that** the said variant comprises a mutation selected from the mutations consisting in the presence, in position 38, of a residue having an acid side chain such as a glutamic acid or an aspartic acid.

2. Variant according to Claim 1, **characterized in that** said variant is derived from the HIV1 TAT protein.

3. Variant according to Claims 1 and 2, comprising aspartic acid in position 38.

4. DNA fragment encoding a TAT protein variant according to one of Claims 1 to 3.

5. Expression cassette comprising a DNA fragment according to Claim 4, placed under the control of appropriate elements permitting its expression.

6. Eukaryotic or prokaryotic cell comprising an expression cassette according to Claim 5.

7. Process for the preparation of a variant according to one of Claims 1 to 3, according to which:
- a eukaryotic or prokaryotic cell according to Claim 6 is cultured in an appropriate culture medium, and
- the variant produced is harvested from the culture medium or from said cell.

8. TAT protein variant obtained using a process according to Claim 7.

9. Variant according to one of Claims 1 to 3, expression cassette according to Claim 5, or cell according to Claim 6, to be used as medicinal product.

10. Pharmaceutical composition intended for the treatment or prevention of a HIV infection which comprises as therapeutic or prophylactic agent a variant according to one of Claims 1 to 3, an expression cassette according to Claim 5 or a cell according to Claim 6.

11. Use of a variant according to one of Claims 1 to 3, of an expression cassette according to Claim 5 or of a cell according to Claim 6, for obtaining a medicinal product intended to inhibit the replication of the HIV virus.
